# EUROPÄISCHE PATENTANMELDUNG

(11) **EP 2 722 402 A1**
(43) Veröffentlichungstag der Anmeldung: **23.04.2014**
(21) Anmeldenummer: 13188707.7
(22) Anmeldetag: 15.10.2013
(51) Int. Cl.: C12Q 1/68, C12N 15/10

(54) **Verfahren zum Trennen, Erfassen oder Anreichern unterschiedlicher DNA-Spezies**

(30) Priorität: 19.10.2012 DE 102012219142
(71) Anmelder: Analytik Jena AG, 07745 Jena (DE)
(72) Erfinder: Osterloh, Dirk, 07743 Jena (DE); Felsmann, Karen, 07743 Jena (DE)
(74) Vertreter: Winter, Brandl, Fürniss, Hübner, Röss, Kaiser, Polte - Partnerschaft

(57) **Zusammenfassung**

Die vorliegende Erfindung betrifft ein Verfahren zum Trennen, Erfassen oder Anreichern von unterschiedlichen DNA-Spezies, die gleichzeitig in einer Probe vorliegen, aufgrund von mit unterschiedlicher Häufigkeit auftretenden nichtmethylierten CpG-Dinukleotiden, innerhalb jeder vorliegenden DNA-Spezies, wobei ein an nichtmethylierte CpG-Motive bindendes Protein an einem Trägermaterial immobilisert wird und die an das immobilisierte Protein gebundene DNA mit einem Elutionsmittelgradienten eluiert wird, wobei bestimmte Konzentrationsbereiche des eingesetzten Elutionsmittels mit bestimmten CpG-Dinukleotid-Häufigkeiten in den vorliegenden DNA-Spezies korrelieren, so dass während der Elution unterschiedliche Fraktionen von DNA-Spezies mit unterschiedlichen Häufigkeiten an CpG-Dinukleotiden erhalten werden.

SEQUENZPROTOKOLL

<110> Analytik Jena AG

<220> Verfahren zum Trennen, Erfassen oder Anreichern von unterschiedlichen
DNA-Spezies

<130> AJ0308

<150> DE10 2012 219142.9

<151> 2012-10-19

<210> 1
<211> 2976
<212> RNA
<213> Homo sapiens

<220>
<221> source
<222> 1..2976
<223> mRNA NM_001101654.1

<400> 1
ggaaagagtg gtggcaggtg aagtcggaga cgacagagga actggtttcc tccgccccgc      60

aaggcacaca gcctgccgac gccccattaa tacatgtgga aggggaaaga gactgaatgg     120

aggaatgaat acaacttgat ccaggtcgtg cttcggaagc ggtcacttta cctgtgaacc     180

tctctgcctg acaaacgggc aatgtacgga atcaaccacc aagatggcgg cgcccgtgaa     240

gaatccgcaa ttaggtcgcc gtcatatgtc gcctaggaac gtacggaatt cgacccacgt     300

acggaatcgg attccaagat gacggcatct atgaggaagt cacgcagtag gtgcagccat     360

gttgcctgta cgtcgaggcc gtacaagcag ccgccgtacg gactctactg acaaggtggc     420

ggcgccctcg ggaaagccac attagagcgc ggccatgttc ccggcgaaca tatggattcg     480

gccaccatac ggatacgata agcaagatgg cggcgcctga ggggtcttgg gggctctagg     540

ccggccacct actggtttgc agcggagacg acgcatgggg cctgcgcaat aggagtacgc     600

tgcctgggag gcgtgactag aagcggaagt agttgtgggc gcctttgcaa ccgcctggga     660

cgccgccgag tggtctgtgc aggttcgcgg gtcgctggcg ggggtcgtga gggagtgcgc     720

cgggagcgga gatatggagg gagatggttc agacccagag cctccagatg ccggggagga     780

cagcaagtcc gagaatgggg agaatgcgcc catctactgc atctgccgca aaccggacat     840

caactgcttc atgatcgggt gtgacaactg caatgagtgg ttccatgggg actgcatccg     900

gatcactgag aagatggcca aggccatccg ggagtggtac tgtcgggagt gcagagagaa     960

agaccccaag ctagagattc gctatcggca caagaagtca cgggagcggg atggcaatga    1020

gcgggacagc agtgagcccc gggatgaggg tggagggcgc aagaggcctg tccctgatcc    1080

agacctgcag cgccgggcag ggtcagggac aggggttggg gccatgcttg ctcggggctc    1140

tgcttcgccc cacaaatcct ctccgcagcc cttggtggcc acacccagcc agcatcacca    1200

gcagcagcag cagcagatca aacggtcagc ccgcatgtgt ggtgagtgtg aggcatgtcg 1260

gcgcactgag gactgtggtc actgtgattt ctgtcgggac atgaagaagt tcgggggccc 1320

caacaagatc cggcagaagt gccggctgcg ccagtgccag ctgcgggccc gggaatcgta 1380

caagtacttc ccttcctcgc tctcaccagt gacgccctca gagtccctgc caaggccccg 1440

ccggccactg cccacccaac agcagccaca gccatcacag aagttagggc gcatccgtga 1500

agatgagggg gcagtggcgt catcaacagt caaggagcct cctgaggcta cagccacacc 1560

tgagccactc tcagatgagg acctacctct ggatcctgac ctgtatcagg acttctgtgc 1620

aggggccttt gatgaccatg gcctgccctg gatgagcgac acagaagagt ccccattcct 1680

ggaccccgcg ctgcggaaga gggcagtgaa agtgaagcat gtgaagcgtc gggagaagaa 1740

gtctgagaag aaggtgatgg agaggaagga ggagcgatac aagcggcatc ggcagaagca 1800

gaagcacaag gataaatgga aacacccaga gagggctgat gccaaggacc ctgcgtcact 1860

gccccagtgc ctggggcccg gctgtgtgcg ccccgcccag cccagctcca agtattgctc 1920

agatgactgt ggcatgaagc tggcagccaa ccgcatctac gagatcctcc cccagcgcat 1980

ccagcagtgg cagcagagcc cttgcattgc tgaagagcac ggcaagaagc tgctcgaacg 2040

cattcgccga gagcagcaga gtgcccgcac tcgccttcag gaaatggaac gccgattcca 2100

tgagcttgag gccatcattc tacgtgccaa gcagcaggct gtgcgcgagg atgaggagag 2160

caacgagggt gacagtgatg acacagacct gcagatcttc tgtgtttcct gtgggcaccc 2220

catcaaccca cgtgttgcct tgcgccacat ggagcgctgc tacgccaagt atgagagcca 2280

gacgtccttt gggtccatgt accccacacg cattgaaggg gccacacgac tcttctgtga 2340

tgtgtataat cctcagagca aaacatactg taagcggctc caggtgctgt gccccgagca 2400

ctcacgggac cccaaagtgc cagctgacga ggtatgcggg tgcccccttg tacgtgatgt 2460

ctttgagctc acgggtgact tctgccgcct gcccaagcgc cagtgcaatc gccattactg 2520

ctgggagaag ctgcggcgtg cggaagtgga cttggagcgc gtgcgtgtgt ggtacaagct 2580

ggacgagctg tttgagcagg agcgcaatgt gcgcacagcc atgacaaacc gcgcgggatt 2640

gctggccctg atgctgcacc agacgatcca gcacgatccc ctcactaccg acctgcgctc 2700

cagtgccgac cgctgagcct cctggcccgg accccttaca ccctgcattc cagatggggg 2760

agccgcccgg tgcccgtgtg tccgttcctc cactcatctg tttctccggt tctccctgtg 2820

cccatccacc ggttgaccgc ccatctgcct ttatcagagg gactgtcccc gtcgacatgt 2880

tcagtgcctg gtggggctgc ggagtccact catccttgcc tcctctccct gggttttgtt 2940

aataaaattt tgaagaaacc aaggaaaaaa aaaaaa 2976

<210> 2

<211> 2964
<212> RNA
<213> Homo sapiens

<220>
<221> source
<222> 1..2964
<223> mRNA NM_014593.3

<400> 2
ggaaagagtg gtggcaggtg aagtcggaga cgacagagga actggtttcc tccgccccgc      60

aaggcacaca gcctgccgac gccccattaa tacatgtgga aggggaaaga gactgaatgg     120

aggaatgaat acaacttgat ccaggtcgtg cttcggaagc ggtcacttta cctgtgaacc     180

tctctgcctg acaaacgggc aatgtacgga atcaaccacc aagatggcgg cgcccgtgaa     240

gaatccgcaa ttaggtcgcc gtcatatgtc gcctaggaac gtacggaatt cgacccacgt     300

acggaatcgg attccaagat gacggcatct atgaggaagt cacgcagtag gtgcagccat     360

gttgcctgta cgtcgaggcc gtacaagcag ccgccgtacg gactctactg acaaggtggc     420

ggcgccctcg ggaaagccac attagagcgc ggccatgttc ccggcgaaca tatggattcg     480

gccaccatac ggatacgata agcaagatgg cggcgcctga ggggtcttgg gggctctagg     540

ccggccacct actggtttgc agcggagacg acgcatgggg cctgcgcaat aggagtacgc     600

tgcctgggag gcgtgactag aagcggaagt agttgtgggc gcctttgcaa ccgcctggga     660

cgccgccgag tggtctgtgc aggttcgcgg gtcgctggcg ggggtcgtga gggagtgcgc     720

cgggagcgga gatatggagg gagatggttc agacccagag cctccagatg ccggggagga     780

cagcaagtcc gagaatgggg agaatgcgcc catctactgc atctgccgca aaccggacat     840

caactgcttc atgatcgggt gtgacaactg caatgagtgg ttccatgggg actgcatccg     900

gatcactgag aagatggcca aggccatccg ggagtggtac tgtcgggagt gcagagagaa     960

agaccccaag ctagagattc gctatcggca caagaagtca cgggagcggg atggcaatga    1020

gcgggacagc agtgagcccc gggatgaggg tggagggcgc aagaggcctg tccctgatcc    1080

agacctgcag cgccgggcag ggtcagggac aggggttggg gccatgcttg ctcggggctc    1140

tgcttcgccc cacaaatcct ctccgcagcc cttggtggcc acacccagcc agcatcacca    1200

gcagcagcag cagcagatca aacggtcagc ccgcatgtgt ggtgagtgtg aggcatgtcg    1260

gcgcactgag gactgtggtc actgtgattt ctgtcgggac atgaagaagt tcgggggccc    1320

caacaagatc cggcagaagt gccggctgcg ccagtgccag ctgcgggccc gggaatcgta    1380

caagtacttc ccttcctcgc tctcaccagt gacgccctca gagtccctgc caaggccccg    1440

ccggccactg cccacccaac agcagccaca gccatcacag aagttagggc gcatccgtga    1500

agatgagggg gcagtggcgt catcaacagt caaggagcct cctgaggcta cagccacacc    1560

tgagccactc tcagatgagg acctacctct ggatcctgac ctgtatcagg acttctgtgc    1620

aggggccttt gatgaccatg gcctgccctg gatgagcgac acagaagagt ccccattcct 1680

ggaccccgcg ctgcggaaga gggcagtgaa agtgaagcat gtgaagcgtc gggagaagaa 1740

gtctgagaag aagaaggagg agcgatacaa gcggcatcgg cagaagcaga agcacaagga 1800

taaatggaaa cacccagaga gggctgatgc caaggaccct gcgtcactgc cccagtgcct 1860

ggggcccggc tgtgtgcgcc ccgcccagcc cagctccaag tattgctcag atgactgtgg 1920

catgaagctg gcagccaacc gcatctacga gatcctcccc cagcgcatcc agcagtggca 1980

gcagagccct tgcattgctg aagagcacgg caagaagctg ctcgaacgca ttcgccgaga 2040

gcagcagagt gcccgcactc gccttcagga aatggaacgc cgattccatg agcttgaggc 2100

catcattcta cgtgccaagc agcaggctgt gcgcgaggat gaggagagca acgagggtga 2160

cagtgatgac acagacctgc agatcttctg tgtttcctgt gggcacccca tcaacccacg 2220

tgttgccttg cgccacatgg agcgctgcta cgccaagtat gagagccaga cgtcctttgg 2280

gtccatgtac cccacacgca ttgaaggggc cacacgactc ttctgtgatg tgtataatcc 2340

tcagagcaaa acatactgta agcggctcca ggtgctgtgc cccgagcact cacgggaccc 2400

caaagtgcca gctgacgagg tatgcgggtg cccccttgta cgtgatgtct ttgagctcac 2460

gggtgacttc tgccgcctgc ccaagcgcca gtgcaatcgc cattactgct gggagaagct 2520

gcggcgtgcg gaagtggact tggagcgcgt gcgtgtgtgg tacaagctgg acgagctgtt 2580

tgagcaggag cgcaatgtgc gcacagccat gacaaaccgc gcgggattgc tggccctgat 2640

gctgcaccag acgatccagc acgatcccct cactaccgac ctgcgctcca gtgccgaccg 2700

ctgagcctcc tggcccggac cccttacacc ctgcattcca gatgggggag ccgcccggtg 2760

cccgtgtgtc cgttcctcca ctcatctgtt tctccggttc tccctgtgcc catccaccgg 2820

ttgaccgccc atctgccttt atcagaggga ctgtccccgt cgacatgttc agtgcctggt 2880

ggggctgcgg agtccactca tccttgcctc ctctccctgg gttttgttaa taaaattttg 2940

aagaaaccaa ggaaaaaaaa aaaa 2964

<210> 3
<211> 570
<212> DNA
<213> Homo sapiens

<220>
<221> source
<222> 1..570
<223> other DNA CXXC1-Insert (BamHI/EcoRI) into pET28a

<400> 3
ggatccggtg gagggcgcaa gaggcctgtc cctgatccaa acctgcagcg ccgggcaggg 60

tcagggacag gggttggggc catgcttgct cggggctctg cttcgcccca caaatcctct 120

ccgcagccct tggtggccac acccagccag catcaccagc agcagcagca gcagatcaaa 180

cggtcagccc gcatgtgtgg tgagtgtgag gcatgtcggc gcactgagga ctgtggtcac 240

tgtgatttct gtcgggacat gaagaagttc gggggcccca acaagatccg gcagaagtgc 300

cggctgcgcc agtgccagct gcgggcccgg gaatcgtaca agtacttccc ttcctcgctc 360

tcaccagtga cgccctcaga gtccctgcca aggccccgcc ggccactgcc cacccaacag 420

cagccacagc catcacagaa gttagggcgc atccgtgaag atgagggggc agtggcgtca 480

tcaacagtca aggagcctcc tgaggctaca gccacacctg agccactctc agatgaggac 540

ctacctctaa gcttaattag ctgagaattc 570

<210> 4
<211> 219
<212> PRT
<213> Homo sapiens

<400> 4
Met Gly Ser Ser His His His His His His Ser Ser Gly Leu Val Pro
1               5                   10                  15
Arg Gly Ser His Met Ala Ser Met Thr Gly Gly Gln Gln Met Gly Arg
            20                  25                  30
Gly Ser Gly Gly Gly Arg Lys Arg Pro Val Pro Asp Pro Asn Leu Gln
        35                  40                  45
Arg Arg Ala Gly Ser Gly Thr Gly Val Gly Ala Met Leu Ala Arg Gly
    50                  55                  60
Ser Ala Ser Pro His Lys Ser Ser Pro Gln Pro Leu Val Ala Thr Pro
65                  70                  75                  80
Ser Gln His His Gln Gln Gln Gln Gln Gln Ile Lys Arg Ser Ala Arg
                85                  90                  95
Met Cys Gly Glu Cys Glu Ala Cys Arg Arg Thr Glu Asp Cys Gly His
            100                 105                 110
Cys Asp Phe Cys Arg Asp Met Lys Lys Phe Gly Gly Pro Asn Lys Ile
        115                 120                 125
Arg Gln Lys Cys Arg Leu Arg Gln Cys Gln Leu Arg Ala Arg Glu Ser
    130                 135                 140
Tyr Lys Tyr Phe Pro Ser Ser Leu Ser Pro Val Thr Pro Ser Glu Ser
145                 150                 155                 160
Leu Pro Arg Pro Arg Arg Pro Leu Pro Thr Gln Gln Gln Pro Gln Pro
                165                 170                 175
Ser Gln Lys Leu Gly Arg Ile Arg Glu Asp Glu Gly Ala Val Ala Ser
            180                 185                 190
Ser Thr Val Lys Glu Pro Pro Glu Ala Thr Ala Thr Pro Glu Pro Leu
        195                 200                 205
Ser Asp Glu Asp Leu Pro Leu Ser Leu Ile Ser
    210                 215

## Beschreibung

Die vorliegende Erfindung betrifft ein in vitro Verfahren zum Trennen, Erfassen oder Anreichern von unterschiedlichen DNA-Spezies, die gleichzeitig in einer Probe vorliegen, gemäß Anspruch 1.

Nukleinsäureamplifikationstechniken (NAT wie zum Beispiel Polymerase-Kettenreaktion, PCR) erlauben eine hochsensitive und hochspezifische Erfassung von mikrobiellen Nukleinsäuren und könnten somit ein ausgezeichnetes Werkzeug zur Verbesserung der Bestimmung und Identifizierung von Bakterien, Pilzen und Viren sein. In der Human- wie auch in der Veterinärmedizin stellt sich das Problem für klinische Proben, dass die zu diagnostischen Zwecken zu erfassenden Mikroorganismen normalerweise nur in sehr geringer Zahl vorkommen und somit nur wenig Target DNA-Moleküle innerhalb der Proben vorhanden sind.

Die Situation wird noch dadurch erschwert, dass die typischen klinischen Proben wie Gewebe- oder Blutproben einen hohen DNA Background enthalten, der durch die Zellen des Wirts hervorgerufen wird, wobei die DNA des Wirtes typischerweise in einem mehrere Zehnerpotenzen umfassenden Überschuss vorliegt.

Daher benötigt man für leistungsfähige mikrobielle Bestimmungssysteme, die auf Nukleinsäureamplifikationstechniken beruhen wirksame und standardisierte Verfahren, die vor der eigentlichen Analyse und/oder Bestimmung durchgeführt werden (Voranalytikprozeduren).

Effektive Voranalytikprozeduren müssen die Elimination einer Vielzahl von inhibierenden und interferierenden chemischen Verbindungen, die im Allgemeinen in biologischen Proben vorliegen wie z. B. Salze, Proteine, Lipide, Metaboliten von Pharmaka und kompetitive Nukleinsäuren, bewältigen können. Im Stand der Technik gibt es eine Reihe von Präparationstechniken, die es erlauben, Salze, Proteine, Lipide, organische Verbindungen und RNA abzutrennen. Diese Techniken schließen unter anderem Präzipitation, Protease- oder Ribonuklease-Verdaus, Flüssig- und Festphasenextraktion ein. Allerdings ist bis heute keine effektive Trennung von relevanten bakteriellen oder pilzlichen DNAs von damit interferierenden eukaryotischen DNAs verfügbar.

Zwar können prokaryotische und eukaryotische DNAs grundsätzlich aufgrund ihrer typischen Unterschiede in Molekulargewichten, Schmelzeigenschaften und epigenetischen Eigenschaften getrennt werden, jedoch stellt dies einen sehr großen apparativen Aufwand dar, der zudem noch eine lange Zeit sowie ausreichende Mengen an eukaryotischer DNA benötigt.

Eine solche Trennung von eukaryotischer und prokaryotischer DNA wäre jedoch aus analytischer Sicht wünschenswert, weil dann eine sicherere und sensitivere Feststellung der DNA aus Mikroorganismen möglich wäre.

Um sich diesem Ziel der Trennung von prokaryotischer und eukaryotischer DNA anzunähern, können die unterschiedlichen Methylierungsmuster, die für prokaryotische und eukaryotische DNA, insbesondere genomische Säuger DNA typisch sind, hilfreich sein.

In diesem Zusammenhang ist es insbesondere bekannt, dass 5-Methylcytosin, eine sogenannte epigenetische Modifikation oder posttranslationale Modifikation der Nukleobase Cytosin, lediglich in genomischer DNA von Eukaryonten auftritt, praktisch jedoch nicht in Prokaryonten.

Die DNA-Methylierung in Eukaryonten beruht auf einer Substitution eines H-Atoms in der 5-Position des Cytosinpyrimidinrings durch eine Methylgruppe.

Es ist bekannt, dass die DNA-Methylierung eine zentrale Rolle bei der normalen Organismenentwicklung und bei der zellulären Differenzierung bei höheren Organismen spielt. Die DNA-Methylierung ändert stabil das Genexpressionsmuster in Zellen oder erniedrigt die Genexpression und bildet die Basis für die Ausbildung der Chromatinstruktur. Aus dem Stand der Technik ist es ebenfalls bekannt, dass die DNA-Methylierung eine zentrale Rolle bei der Entwicklung von Neoplasien eine große Rolle spielt (Jaenisch und Bird, 2003: Epigenetic regulation of gene expression: how the genome integrates intrinsic and environmental signals; Nature Genetics 33 Suppl: 245-254).

### DNA-Methylierung bei Säugern

Die Substitution eines Wasserstoffatoms in der 5-Position des Cytosinrings durch eine Metylgruppe in CpG-Dinukleotiden stellt im Säugergenom die hauptsächlich auftretende epigenetische Veränderung dar. Dies wurde bisher für jedes untersuchte Mitglied der Vertebraten bestätigt.

Insbesondere sind zwischen 60% und 90% sämtlicher CpG-Nukleotide in Mammaliern methyliert (Ehrlich et al, 1982 Amount and distribution of 5-methylcytosine in human DNA from different types of tissues of cells. Nucleic Acids Research 10 (8): 2709-2721 und Tucker KL, 2001, Methylated cytosine and the brain: a new base for neuroscience. Neuron 30 (3): 649-652). Aufgrund ihrer chemischen Struktur können methylierte Cytosinnukleobasen spontan deaminieren, um Thyminnukleobasen zu bilden. Demzufolge mutieren methylierte CpG-Dinukleotide regelmäßig zu TpG-Dinukleotiden. Ein Hinweis für das Vorliegen derartiger Mutationen ist gegeben durch die Unterrepräsentation von CpG-Dinukleotiden im Humangenom, da sie dort lediglich mit etwa 21 % der erwarteten Häufigkeit auftreten (International Human Genome Sequencing Consortium, 2001 "Initial sequencing and analysis of the human genome. Nature 409 (6822): 860-921"). Andererseits führt die spontane Deaminierung von nicht methylierten Cytosinnukleobasen zur Umwandlung derselben zu Uracil, eine Mutation, welche jedoch schnell von der Zelle erkannt wird und repariert wird.

Nichtmethylierte CpGs sind häufig in sogenannten Clustern gruppiert, die man CpG-Inseln nennt. Sie liegen in den 5'-regulatorischen Bereichen vieler Gene. In vielen Krankheitsprozessen, wie beispielsweise Krebs, zeigen die CpG-Inseln in Genpromotoren abnorme Hypermethylierungsmuster, welche zu transkriptionalem Silencing führen, was im Anschluss an die Zellteilung an die Tochterzelle vererbt werden kann.

### DNA-Methylierung bei Pilzen

Pilze zeigen typischerweise sehr niedrige Niveaus an Cytosin-Methylierung. Das Ausmaß der Methylierung variiert zwischen 0,1 und 5% speziesabhängig (Antequera et al. 1984 DNA methylation in the fungi. J. Biol. Chem. 259 (13): 8033-8036).

Insbesondere scheinen diese Methylierungswerte auch noch innerhalb derselben Spezies zu variieren (siehe Binz et al., 1998 "A comparison of DNA methylation levels in selected isolates of higher fungi. Mycologia (Mycological Society of America) 90 (5): 785-790").

### DNA-Methyllerung bei Bakterien

Während eukaryontische DNA-Methylierung ausschließlich Cytosinreste innerhalb der DNA betrifft und spezifisch für CpG-Motive ist, können bei Bakterien sowohl Adeninals auch Cytosinreste methyliert werden.

Bislang wurde eine Reihe von DNA Methyltransferasen (DNA-MTasen) entdeckt, welche die Cytosinmethylierung im unterschiedlichen Sequenzkontext katalysieren (Noyer-Weidner und Trautner, 1993 "Methylation of DNA in prokaryotes. EXS 64, 39-108"). So enthalten beispielsweise die meisten Stämme von E. coli sequenzspezifische DNA-Methylasen:
Dam-Methylase: Methylierung an der N⁶-Stellung oder Position des Adenins in der Sequenz GATC
Dcm-Methylase: Methylierung an der C⁵-Position des Cytosins in der Sequenz CCAGG und CCTGG
EcoKI-Methylase: Methylierung des Adenins in den Sequenzen AAC (N⁶A) GTGC und GCAC (N⁶A) GTT.

Die hauptsächliche Funktion der DNA-Methylierung in Bakterien ist es einen Mechanismus zur Verfügung zu stellen, welche die Bakterienzellen vor dem Eigenverdau sowie dem Eindringen von Fremd DNA zu schützen. Bakterielle Restriktionsendonukleasen können zwischen endogener und Fremd DNA anhand ihres Methylierungsmusters unterscheiden. Beispielsweise wird von Phagen eingeführte DNA, welche nicht durch die der Wirtszelle entsprechende Methylierung geschützt ist, durch Spaltung eliminiert (Noyer-Weidner und Trautner, 1993 "Methylation of DNA in prokaryotes. EXS 64, 39-108").

Im Stand der Technik ist bekannt, dass der GC-Gehalt in Genomen unterschiedlicher Spezies, insbesondere bakteriellen Spezies stark variiert. Innerhalb der Domäne der Bakterien wurden GC-Gehalte zwischen 25 und 75% gemessen (siehe Hill, L.R. 1966. An Index to deoxyribonucleic acid base compositions of bacterial species. J. Gen. Microbiol. 44:419-437). Aufgrund der unterschiedlichen GC-Gehalte von Spezies innerhalb einer Gattung sowie der phylogenetischen Unterschiede gab es im Stand der Technik auch Vorschläge, Bakterien anhand ihres GC-Gehaltes zu klassifizieren (Siehe Wayne et al, 1987 "Report of the ad hoc committee on reconciliation of approaches to bacterial systematic. International journal of systematic bacteriology 37 (4): 463-4").

Eine große Diversität der GC-Gehalte wurde mit Werten von 26-70% auch bei Pilzen (siehe Storck, R and C.J. Alexopoulos,1970. Desoxyribonucleic acid of fungi. Bacteriol.Rev. 34(2):126) mit Werten von 22-68% bei Protozoen und 37-68% bei Algen (siehe Mandel, M. 1967 Nucleic acids of protozoa, p. 541-572. In Florkin, M., Scheer, B,T" and G.W. Kidder, Chemical zoology, vol.I. Academic Press Inc., New York) und mit Werten von 35-71% bei Cyanobakterien (siehe Edleman, M., Swinton,D., Schiff, J.A., Epstein, H.T., and B. Zeldin, 1967. Deoxyribonucleic acid of the blue-green algae (cyanophyta). Bacteriol.Rev. 31:315-331.) festgestellt.

Im Folgenden sind in Tabelle 1 die GC-Gehalte ausgewählter Modellorganismen wiedergegeben.

**Tabelle 1**

| **Species** | **phylogenetische Klassifikation** | **GC-Gehalt*** |
|---|---|---|
| Streptomyces coelicolor | Actinobacterium | 72% |
| Myxococcus xanthus | Deltaproteobacterium | 68% |
| Halobacterium sp. | Archaeon | 67% |
| Saccharomyces cerevisiae | Ascomycete (fungus) | 38% |
| Arabidopsis thaliana (Ackerschmalwand)** | Blütenpflanze | 36% |
| Methanosphaera stadtmanae | Archaeon | 27% |
| Plasmodium falciparum | Protozoon | ~20% |

| | | |
|---|---|---|
| *Der GC-Gehalt wird im Allgemeinen als ein Prozentwert ausgedrückt. Der GC-Gehaltprozentsatz wird berechnet als [G+C / A+T+G+C] x 100. **Schotenkresse, Gänseranke [EN: Thale Cress] | | |

Beispielsweise wird die Actinobacteria im Taxonomiebrowser des National center for biotechnology information [NCBI] charakterisiert. Bei Streptomyces coelicolor A3 (2) beträgt der GC-Gehalt 72% gemäß den Gesamtgenomdaten des NCBI. Der GC-Gehalt von Hefe (Saccharomyces cerevisiae) beträgt 38 % gemäß der Gesamtgenomdaten des NCBI und der GC-Gehalt eines weiteren häufig benutzten Modellorganismus, nämlich der Ackerschmalwand (Arabidopsis thaliana) beträgt 36% gemäß den Gesamtgenomdaten des NCBI.

Dem Fachmann ist es wohl bekannt, dass es aufgrund der Natur des genetischen Codes für einen Organismus praktisch unmöglich ist, ein Genom mit einem GC-Gehalt von entweder 0% oder 100% aufweisen zu können. Jedoch ist eine Spezies mit einem extrem niedrigen GC-Gehalt der Malariaerreger Plasmodium falciparum (GC ~20% gemäß dem Gesamtgenomdaten aus Plasmodium falciparum des NCBI und es hat sich in der wissenschaftlichen Gemeinschaft eingebürgert, derartige Organismen eher als "AT-reich" anzusehen, als diese mit "GC-arm" zu bezeichnen (siehe Musto et al., 1997 "Compositional constraints in the extremely GC-poor genome of Plasmodium falciparum. Mem. Inst. Oswaldo Cruz 92 (6): 835-41 ").

Es ist wichtig zu wissen, dass GC-Verhältnisse innerhalb eines Genoms merklich variieren können. So variiert beispielsweise der GC-Gehalt der menschlichen DNA stark über das Genom, beginnend mit 30% bis 60% und diese GC-Verhältnisvariationen innerhalb des Genoms von komplexeren Organismen führen daher zu einer mosaikartigen Formation mit Inselregionen, sogenannten Isochoren (siehe Bernardi, 2000 "Isochores and the evolutionary genomics of vertebrates". Gene 241 (1): 3-17)").

GC-reiche Isochore enthalten viele proteinkodierende Gene und daher kann die Bestimmung der Verhältnisse dieser spezifischen Bereiche zum Kartieren genreicher Bereiche des Genoms beitragen (Vergleiche Sumner et al, 1993 "The distribution of genes on chromosomes: a cytological approach." J. Mol. Evol. 37 (2): 117-22; Aïssani und Bernardi, 1991 "CpG islands, genes and isochores in the genomes of vertebrates." Gene 106 (2): 185-95).

Wie bereits Eingangs erwähnt, können derartige GC-reiche Bereiche und insbesondere die CpG-Inseln am Cytosin methyliert oder nichtmethyliert sein.

Im Wesentlichen ist festzuhalten, dass Bakterien nichtmethylierte CpG Motive enthalten, während insbesondere Human-DNA methylierte CpG Motive enthält.

Aus dem Stand der Technik der EP 1 400 589 B1 sowie der WO 2004/033683 A1 ist bekannt an nichtmethylierte CpG-DNA bindende Proteine zum Anreichern und/oder Abtrennen von bakterieller DNA von humaner DNA in Proben, in welchen ein starker Überschuss an Human-DNA vorliegt, zu verwenden.

Die selektive Bindung von spezifischen Proteinen an nicht-methylierte CpG-DNA wird vermittelt durch die CXXC Proteindomäne (zf-CXXC; Pfam PF02008). Bislang wurden im Stand der Technik eine Reihe von Proteinen, welche die CXXC Domäne enthalten, beschrieben. Dies sind beispielsweise das an die methyl-CpG bindende Domäne bindende Protein (MBD1) (Vergleiche Cross et al., 1997 "A component of the transcriptional repressor MeCP1 shares a motif with DNA methyltransferase and HRX proteins. Nat Genet 16: 256-259"), DNA Methyltransferase 1 (DNMT1) (siehe Bestor und Verdine, 1994 "DNA methyltransferases. Curr Opin Cell Biol 6: 380-389"), die hauptsächliche DNA Reparatur DNA Methyltransferase (FBXL11), welche kürzlich als Histon-Demethylase charakterisiert wurde, welche spezifisch das Histon H3 Aminolysin 36 demethyliert (Tsukada et al., 2006 "Histone demethylation by a family of JmjC Domäne-containing proteins. Nature 439: 811-816") sowie das CpG-bindende Protein (CGBP), eine Komponente des Säuger Set1 H3-H4 Methyltransferase-Komplexes (Lee und Skalnik, 2005 "CpG-binding protein (CXXC finger protein 1) is a component of the mammalian Set1 histone H3-Lys4 methyltransferase complex, the analogue of the yeast Set1/COMPASS complex. J Biol Chem 280: 41725-41731 ").

Die cysteinreiche CXXC Domäne wird in einer Reihe von Chromatin-assoziierten Proteinen gefunden und ist verantwortlich für die spezifische Bindung des Proteins an nichtmethylierte CpG-Dinukleotide. Die CXXC Domäne weist zwei CGXCXXC-Repeats auf. Soweit bekannt, enthält die CXXC Domäne - ohne hieran gebunden zu sein - acht konservierte Cysteinreste, welche zwei Zinkionen binden können. Die molekulare Grundlage der Erkennung nichtmethylierter CpG DNA wurde insbesondere von Allen et al., 2006 "Solution structure of the nonmethyl-CpG-binding CXXC Domäne of the leukaemia-associated MLL histone methyltransferase; The EMBO Journal 25, 4503-4512" untersucht, welche die Raumstruktur der CXXC Domäne des humanen CXXC Proteins MLL durch multidimensionale NMR Spektroskopie bestimmt haben. Gemäß ihren Arbeiten weist die CXXC Domäne eine Falte auf, in welcher zwei Zinkionen tetraedrisch durch vier konservierte Cysteinliganden, die von zwei CGXCXXC-Motiven und zwei distalen Cysteinresten zur Verfügung gestellt werden, koordiniert sind. Allen et al., 2006 haben auch die Bindungsaffinität der CXXC Domäne gegenüber DNA-Molekülen, welche ein zentral gelegenes nicht-methyliertes CpG-Dinukleotid aufweisen, gemessen. Sie haben herausgefunden, dass die CXXC Domäne eine 12-mer DNA mit einem zentralen CpG-Motiv mit einer Bindungsaffinität von K_{d} von 4,3 µM ± 0,4 µM bindet. Keine Bindung wurde für dasselbe DNA Molekül beobachtet, wenn es ein methyliertes zentrales CpG-Motiv enthielt.

Ausgehend vom Stand der Technik der EP 1 400 589 B1 ist es daher Aufgabe der vorliegenden Erfindung ein Verfahren zum Trennen, Erfassen oder Anreichern von unterschiedlichen DNA-Spezies zur Verfügung zu stellen, welches beispielsweise eine einfache und zuverlässige taxonomische Bestimmung bakterieller und pilzlicher Mikroorganismen ermöglicht.

Diese Aufgabe wird durch ein Verfahren mit den Merkmalen des Anspruchs 1 gelöst.

Insbesondere betrifft die vorliegende Erfindung ein in vitro Verfahren zum Trennen, Erfassen oder Anreichern von unterschiedlichen DNA-Spezies, die gleichzeitig in einer Probe vorliegen, aufgrund von mit unterschiedlicher Häufigkeit auftretenden nichtmethylierten CpG-Dinukleotiden, innerhalb jeder vorliegenden DNA-Spezies, wobei nichtmethylierte CpG-Dinukleotide solche sind, welche keine Methylgruppe in Position 5 des Cytosins aufweisen, wobei
die DNA, welche mehrere unterschiedliche DNA-Spezies enthält, aus der Probe isoliert wird und eine wässrige Lösung der isolierten DNA hergestellt wird;
die DNA-Lösung mit wenigstens einem an einem Trägermaterial immobilisierten an nichtmethylierte CpG-Dinukleotide bindenden Protein in Kontakt gebracht wird;
unspezifisch gebundene DNA und methylierte DNA ausgewaschen wird; und
das Trägermaterial mit wenigstens einem Elutionsmittel, welches unterschiedliche Konzentrationen aufweist, eluiert wird, wobei bestimmte Konzentrationsbereiche des eingesetzten Elutionsmittels mit bestimmten CpG-Dinukleotid-Häufigkeiten in den vorliegenden DNA-Spezies korrelieren, so dass während der Elution unterschiedliche Fraktionen von DNA-Spezies mit unterschiedlichen Häufigkeiten an GpG-Dinukleotiden erhalten werden, wobei die unterschiedlichen DNA-Spezies aus unterschiedlichen Bakterien und/oder Pilzen stammen.

Mit Hilfe der vorliegenden Erfindung können DNA-Spezies aus unterschiedlichen Bakterien und/oder Pilzen getrennt werden.

Für die Zwecke der vorliegenden Erfindung wird unter dem Begriff CpG-Dinukleotid ein zwei Nukleobasen langer Bereich innerhalb einer DNA verstanden, der die Bestandteile Cytosin-Phosphat-Guanin in Leserichtung vom 5'- zum 3'-Ende enthält und beispielsweise durch folgende Struktur wiedergegeben werden kann:

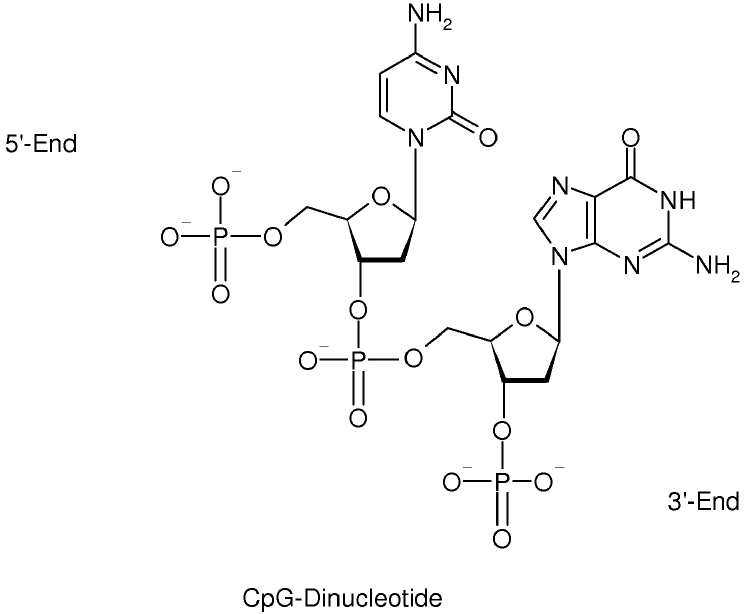

Nichtmethylierte CpG-Dinukleotide sind definitionsgemäß solche, welche keine Methylgruppe in Position 5 des Cytosins aufweisen, also den Nukleobasenrest gemäß folgender chemischer Struktur **nicht** enthalten:

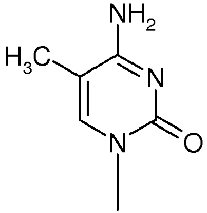

Derartige CpG-Dinukleotide werden auch CpG-Motive genannt.

Ein bevorzugtes erfindungsgemäßes Verfahren ist dadurch gekennzeichnet, dass die unterschiedlichen DNA-Spezies aus taxonomisch unterschiedlichen Mikroorganismen stammen. Bei diesen Mikroorganismen handelt es sich bevorzugt um

Bakterien und/oder Pilze, welche insbesondere ausgewählt sind aus der Gruppe bestehend aus:
den Bakteriengattungen:
   Pseudomonas; Stenotrophomonas; Neisseriaceae; Echerichia; Pseudomonas; Proteus; Acinetobacter; Streptococcus; Staphylococcus; Clostridium;
den Bakterienspezies:
   Pseudomonas aeroginosa, Stenotrophomonas maltophila, Neisseria meningitidis, Echerichia coli, Proteus mirabilis, Acinetobacter baumannii, Streptococcus pneumoniae, Staphylococcus aureus, Clostridium perfringens;
den Pilzgattungen Aspergillus und Saccharomyces; sowie
den Pilzspezies Aspergillus niger und Saccharomyces cerevisiae.

In einer bevorzugten Variante des erfindungsgemäßen Verfahrens wird das Erfassen von unterschiedlichen DNA-Spezies zur Identifikation von Mikroorganismen, insbesondere Bakteriengattungen und/oder Pilzgattungen und/oder Protozoengattungen, und/oder Algengattungen, und/oder Cyanobakteriengattungen und/oder Bakterienspezies und/oder Pilzspezies, und/oder Protozoenspezies, und/oder Algenspezies, und/oder Cyanobakterienspezies verwendet.

Eine ebenfalls bevorzugte Ausführungsform des vorliegenden erfindungsgemäßen Verfahrens liegt darin, dass die Identifikation von Mikroorganismen/Bakterien anhand von erfassten DNA-Spezies in der Probe über folgende Zuordnung von Häufigkeiten des CpG-Dinukleotids innerhalb der erfassten DNA-Spezies erfolgt, ausgedrückt als Kehrwert der Frequenz (•) der CpG-Dinukleotide, wobei die Frequenz die Anzahl der Nukleotide [nt] in einer DNA Spezies bedeutet, auf die statistisch ein CpG-Dinukleotid entfällt:

| **Mikroorganismus-Spezies** | **1/• CpG-Dinukleotide [nt]** |
|---|---|
| Pseudomonas aeruginosa | 36.0-36.4, bevorzugt 36.2 |
| Stenotrophomonas maltophilia | 36.5-36.8, bevorzugt 36.6 |
| Neisseria meningitidis | 42.4-43.2, bevorzugt 42.8 |
| Echerichia coli | 55.1-55.9, bevorzugt 55.5 |
| Proteus mirabilis | 111.5-112.6, bevorzugt 112.1 |
| Acinetobacter baumannii | 120.9-121.9, bevorzugt 121.4 |
| Streptococcus pneumoniae | 144.5-145.5, bevorzugt 145.0 |
| Staphylococcus aureus | 159.2-160.2, bevorzugt 159.7 |
| Clostridium perfringens | 801.7-805.7, bevorzugt 803.7 |

In vorteilhafter Weise kann gemäß der vorliegenden Erfindung als Maß für die Häufigkeit des CpG-Dinukleotids in einer DNA-Spezies der Gehalt an den Nukleobasen Guanin und Cytosin (GC-Gehalt) verwendet werden, ausgedrückt in % als $\left[\frac{mGuanin + mCytosin}{mAdenin + mThymin + mGuanin + mCytosin}\right] \times 100$
wobei m die Masse der jeweiligen Nukleobase bezeichnet.

Eine weitere bevorzugte Ausführungsform des erfindungsgemäßen Verfahrens ist dadurch gekennzeichnet, dass das Trägermaterial mit einer Salzlösung, insbesondere einem Salzgradienten, bevorzugt einem (NH₄)₂CO₃- Gradienten oder einem NaCl-Gradienten mit einer NaCl-Konzentration von 0,01 M bis 1,0 M, insbesondere 0,1 M bis 0,8 M, bevorzugt 0,2 M bis 0,7 M eluiert wird, wobei bestimmte Konzentrationsbereiche einer bestimmten CpG-Häufigkeit und/oder einem bestimmten GC-Gehalt entsprechen, so dass einzelne DNA-Fraktionen mit unterschiedlichen CpG-Häufigkeiten und/oder unterschiedlichen GC-Gehalten erhalten werden; und

Vergleichen der Salzkonzentrationen der einzelnen eluierten DNA-Fraktionen mit der Salzkonzentration von eluierter DNA mit bekannter CpG-Häufigkeit und/oder bekanntem GC-Gehalt aus bekannten Mikroorganismen, um den unbekannten Mikroorganismus anhand seiner CpG-Häufigkeit und/oder seines GC-Gehaltes in der jeweiligen DNA-Fraktion zu identifizieren.

Es ist bevorzugt, die Salzkonzentration der einzelnen DNA-Fraktionen über eine Messung der elektrischen Leitfähigkeit zu erfassen. Dies stellt eine einfache und schnell durchzuführende Messung dar, wofür es im Stand der Technik eine Reihe von Messinstrumenten gibt.

Eine weitere bevorzugte Ausführungsform des erfindungsgemäßen Verfahrens ist dadurch gekennzeichnet, dass das an nichtmethylierte CpG-Dinukleotide bindende Protein ausgewählt wird aus der Gruppe bestehend aus: wenigstens eine CXXC-Domäne enthaltende Proteine; Proteinfragmente, welche wenigstens eine CXXC-Domäne aufweisen, insbesondere humanes nichtmethyl-CpG bindendes Protein CXXC1/CGBP sowie dessen Isoformen 1 und 2 und Fragmente davon, die die CXXC-Domäne enthalten; Fusionsproteine, welche die CXXC-Domäne enthalten, insbesondere Fusionsproteine, welche Sequenzen des humanen nichtmethyl-CpG bindenden Proteins CXXC1/CGBP enthalten.

Insbesondere enthält die CXXC Domäne acht konservierte Cysteinreste, die zwei Zinkionen binden können. Die Domäne ist charakterisiert durch zwei CGXCXXC Sequenzmotive. Die CXXC Domäne bindet an nichtmethylierte CpG Dinukleotide.

Siehe Cross SH, Meehan RR, Nan X, Bird A; , Nat Genet 1997;16:256-259.: A component of the transcriptional repressor MeCP1 shares a motif with DNA methyltransferase and HRX proteins; Bestor TH; , EMBO J 1992;11:2611-2617.: Activation of mammalian DNA methyltransferase by cleavage of a Zn binding regulatory Domäne; sowie Allen MD, Grummitt CG, Hilcenko C, Min SY, Tonkin LM, Johnson CM, Freund SM, Bycroft M, Warren AJ; , EMBO J. 2006;25:4503-4512.: Solution structure of the nonmethyl-CpG-binding CXXC Domäne of the leukaemia-associated MLL histone methyltransferase; und Chao Xu, Chuanbing Bian, Robert Lam, Aiping Dong & Jinrong Min; , Nat. Commun. 2011 ;2:227.: The structural basis for selective binding of nonmethylated CpG islands by the CFP1 CXXC Domäne.

Es ist ferner bevorzugt, dass das an nichtmethylierte CpG-Dinukleotide bindende Protein an seinem N-terminalen und oder an seinem C-terminalen Ende mit einer Aminosäuresequenz fusioniert ist, die eine Affinität für Proteine und Peptide, beispielsweise für Antikörper (c-myc-tag, FLAG-tag), für Streptavidin (Strep-tag), für Calmodulin (Calmodulin-bindendes Oligopeptid) oder Glutathion (Glutathion-S-Transferase-tag) aufweist. Weitere an das nichtmethylierte CpG-Dinukleotide bindende Protein fusionierte Aminosäuresequenzen können eine Bindung an Biotin (BCCP-Tag), Amylose (Maltose binding protein-tag) oder Metallionen vermitteln. Eine bevorzugte Ausführungsform ist die Fusion an eine metallionenbindende Aminosäuresequenz , insbesondere an einen Hisₙ-tag, , wobei n 3 bis 10, bevorzugt 6, ist.

Eine terminale Form der Derivatisierung des an nichtmethylierte CpG-Dinukleotide bindenden Proteins, das heißt seine Modifizierung am N-Terminus und/oder am C-Terminus zum Zweck der kovalenten oder der nichtkovalenten Immobilisierung an Trägermaterialien bietet den Vorteil der Minderung sterischer Probleme bei der Wechselwirkung des Proteins mit Trägermaterialien und bei der Wechselwirkung mit Nukleinsäuren.

Eine ebenfalls bevorzugte Ausführungsform des erfindungsgemäßen Verfahren ist, dass das Trägermaterial ein Metallchelat-bildendes Material ist, insbesondere eine Metallchelat-bildende Agarose, bevorzugt eine nickelionenbindende Agarose, vorzugsweise Sepharose.

Gemäß der im Rahmen der vorliegenden Erfindung gemachten experimentellen Erfahrungen ist es bevorzugt, dass das an nichtmethylierte CpG-Dinukleotide bindende Protein an seinem N-terminalen Ende ein His₆- tag, aufweist, und über Ni²⁺-Kationen an Nickelchelat-bildende Sepharose immobilisiert ist.

Insbesondere verwendet die vorliegende Erfindung humane Proteine, welche an nicht-methylierte CpG-Dinukleotide enthaltende DNA binden. Als eines der bevorzugten Proteine wird das CXXC1/CGBP zur Entwicklung eines präanalytischen Systems verwendet. Selbstverständlich ist dem Fachmann wohl bekannt, dass auch sämtliche Protein-Isoformen wie beispielsweise die CXXC1 Isoform 1 und die CXXC1 Isoform 2 verwendet werden können.

Wie bereits erwähnt ist die CXXC Domäne für die Bindung an nicht-methylierte CpG DNA verantwortlich (siehe Voo et al., 2000 "Cloning of a mammalian transcriptional activator that binds unmethylated CpG motifs and shares a CXXC Domain with DNA methyltransferase, human trithorax, and methyl-CpG binding Domain protein 1. Mol Cell Biol. Mar;20(6):2108-21 ").

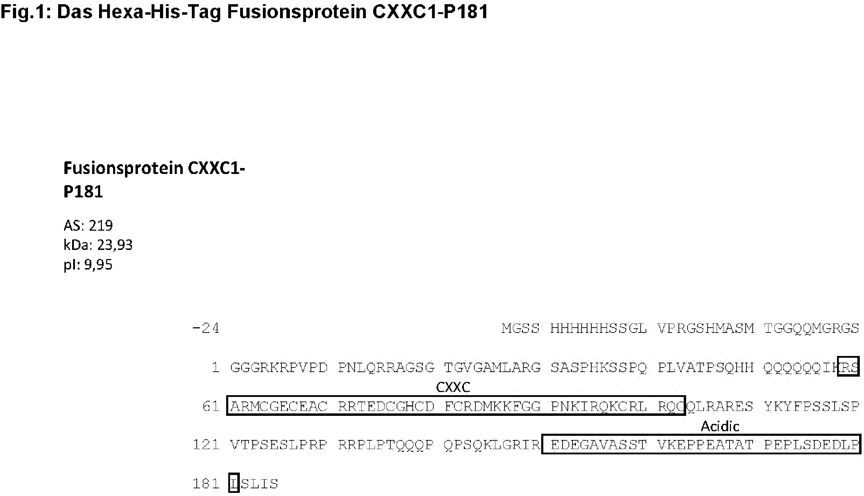

Typischerweise wird nur ein Teil der CXXC1 Sequenz benötigt, zum Beispiel für die Konstruktion eines trunkierten CXXC1-His-tag Fusionsproteins. Der CXXC1-Teil enthält die CXXC Domäne und eine Aminosäuresequenz mit saurem Charakter und wurde beispielsweise an eine Hexa-His-Einheit und Linkersequenz fusioniert, die durch den bakteriellen Proteinexpressionsvektor pET28a(+) kodiert wird. Die Aminosäuresequenz des sich ergebenden Fusionsproteins ist in Figur 1 dargestellt. Hierbei handelt es sich um das sogenannte Hexa-His-Tag Fusionsprotein CXXC1-P181. (vgl. SEQ-ID No 4 sowie cDNA und Isofomen gemäß SEQ-ID No 1 bis 3). Die geladenen Aminosäurereste, die sich im sauren Bereich der Sequenz befinden, vermitteln die Löslichkeit des Proteins in physiologischen Puffern.

In Figur 1 sind funktionelle Domänen umrahmt. Das Fusionsprotein enthält 185 Aminosäuren, die aus der CXXC1-Sequenz stammen, wird jedoch als P181 bezeichnet, weil sich die Anzahl der Aminosäuren auf ein Precursorkonstrukt beziehen.

Im Zusammenhang mit der vorliegenden Erfindung wird ein allgemeines E.coli Expressionssystem verwendet, nämlich das pET28a(+) / E.coli BL21 (DE3)-System. Mit Hilfe dieses Systems kann das Hexa-His-Tagfusionsprotein CXXC1-P181 effizient exprimiert werden. Das rekombinant produzierte Protein erlaubt eine leichte Lösbarkeit unter Verwendung von chaotropen Puffern und kann mit Hilfe des sogenannten On-Column-Refoldings in einen aktiven strukturellen Status überführt werden.

Ein aktives CXXC-haltiges Protein oder dessen relevante Fragmente werden für die Zwecke der vorliegenden Erfindung "LOOXSTER-Protein" genannt. (LOOXSTER ist eine unter der Nummer GM 004601027 eingetragene Gemeinschaftsmarke der Anmelderin SIRS-Lab GmbH, Jena). Beispielhaft bezeichnet LOOXSTER-Protein auch das CXXC1-P181-Protein, welches stellvertretend für die anderen, an nichtmethylierte CpG-Motive bindenden Proteine im Rahmen der vorliegenden Ausführungsbeispiele zur detaillierten Beschreibung und Offenbarung der vorliegenden Erfindung benutzt wird.

Die LOOXSTER-Proteine und insbesondere das CXXC1-P181-Protein, können leicht über ihre N-terminalen His-Tags an unterschiedlichen metallchelatisierenden, festen Trägern immobilisiert werden. Im Zusammenhang mit der vorliegenden Erfindung wird ein LOOXSTER-Protein in seinem immobilisierten Zustand "LOOXSTER-Partikel" genannt.

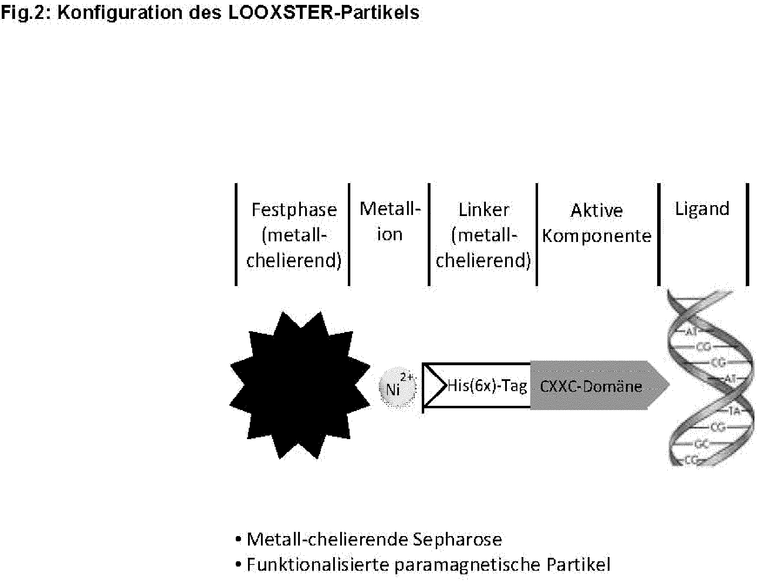

Die schematische Zusammensetzung eines LOOXSTER-Partikels ist in Figur 2 verdeutlicht. Die aktive Komponente und die Chemie der Immobilisierung erlauben nur kleinere Modifikationen. Allerdings kann der feste Träger zusätzliche chemische und/oder physikalische Parameter dem LOOXSTER-System zufügen. Hierzu zählen beispielsweise das Partikelmaterial, die Partikelgröße, die Porosität, die Dichte oder die funktionellen Gruppen, Spacer, Para- oder Ferromagnetismus.

Insbesondere ist das Fusionsprotein CXXC1-P181 über einen N-terminalen und/ oder C-terminalen Hexa-His-Tag auf der Oberfläche von metallchelatisierenden Trägern immobilisiert. Das so entstehende an nicht-methylierte CpG-Motive bindende Partikel kann zusammengesetzt werden unter Verwendung von Trägern unterschiedlicher chemischer und physikalischer Eigenschaften. Aufgrund der sterischen Anforderungen der Wechselwirkung des an nichtmethylierte CpG-Motive bindenden Proteins mit DNA ist eine terminale Immobilisierung des Proteins unter Einbeziehung des N-Terminus und oder des C-Terminus des an nichtmethylierte CpG-Motive bindenden Proteins bevorzugt. Die im Zusammenhang mit der vorliegenden Erfindung verwendete Konfiguration erlaubt eine ausreichende Minderung solcher sterischen Hinderungen.

Die Verwendung des N-Terminus und oder des C-Terminus für die Immobilisierung ist auch geeignet, um sterische Probleme, die die Effizienz dieses Prozesses verringern können, zu minimieren.

Abhängig von den physikalischen Eigenschaften des festen Trägers können die LOOXSTER-Partikel als Batch- oder Kartuschen-/Säulen-basierte Assaysysteme ausgebildet sein. Eine bevorzugte Ausführungsform der vorliegenden Erfindung liegt in metallchelatisierten Agarosederivaten (Sepharosen) mit einer Partikelgröße von etwa 90 µm und paramagnetischen Nickel-chelatisierenden Beads, welche einen Partikeldurchmesser von 2 - 14 µm aufweisen. Ein derartiges System hat sich sowohl für Kartuschen bzw. Säulen als auch für Batchsysteme als vorteilhaft herausgestellt.

Die Wechselwirkung von an nichtmethylierte CpG-Motive bindenden Proteinen mit DNA wurde untersucht mit Hilfe von affinitätschromatographischen Assays. Um dies zu erreichen, wurde beispielsweise ein CXXC1-P181-Protein an einer Nickelsepharose, z. B. Ni-Sepharose 6 FF (GE-Healthcare) mit einer definierten Proteindichte immobilisiert und die so hergestellte Affinitätsmatrix wurde verwendet, um eine 1 ml Chromatographiesäule zu packen. Diese sogenannte LOOXSTER-Säule wurde dann mit einem Chromatographiesystem (z.B. ÄKTA-Purifier; GE-Healthcare) verbunden. Der LOOXSTER Chromatographieassay bestand aus mehreren Schritten: Equilibrierung, Probenauftrag, Auswaschen ungebundenen Probenmaterials, Elution mittels eines linearen Gradienten, Halten eines geeigneten Konzentrationsplateaus, Umkehrgradient und Reequilibrierung.

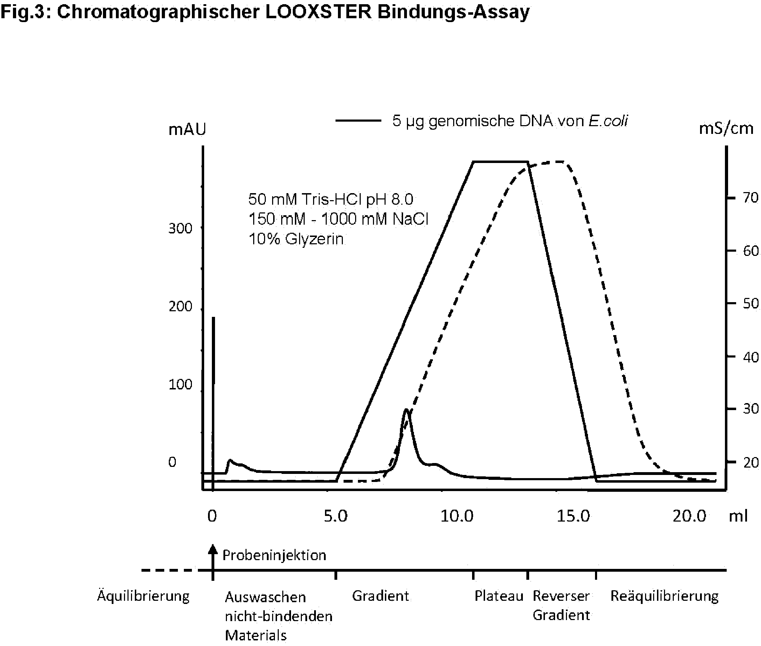

Das Binden und Ablösen einer DNA-Probe von der LOOXSTER-Säule wurde durchgeführt durch Ändern der NaCl Konzentration. UV Absorption bei 260 nm und Leitfähigkeit wurden gemessen und aufgezeichnet. Die Affinität des LOOXSTER-Proteins zu der aufgetragenen DNA Probe unter den gegebenen Bedingungen wurde gemessen als der Leitfähigkeitswert am Maximum der Absorptionspeaks (Vergleiche Fig. 3). Der Assay wurde verwendet, um die Wechselwirkung des LOOXSTER-Systems mit humaner genomischer DNA und bakterieller genomischer DNA aus unterschiedlichen Spezies sowie genomischer DNA von ausgewählten Pilz-Spezies zu testen. Insbesondere wurde der Assay verwendet, um den Einfluss von unterschiedlichen Parametern wie pH-Wert, Methylierung, Häufigkeit der CpG-Motive usw. auf die LOOXSTER-DNA-Interaktion zu bestimmen.

Figur 3 zeigt ein typisches Chromatogramm mit den Schritten des chromatographischen Zyklus'. Die UV-Absorptionskurve zeigt deutlich, dass die DNA-Probe, welche ausschließlich nichtmethylierte CpG Motive enthält, nämlich die genomische DNA aus E.coli praktisch quantitativ an die LOOXSTER-Säule bei einer niedrigen NaCl-Konzentration gebunden wird und bei deutlich höherer NaCl-Konzentration mittels eines linearen Gradienten von der Säule eluiert werden kann,

### Beschreibung der LOOXSTER-Protein/DNA Wechselwirkung

Im vorliegenden Beispiel wurde die Wechselwirkung des LOOXSTER-Proteins mit humanen und bakteriellen genomischen Desoxyribonukleinsäuren unter Verwendung des oben beschriebenen Assays untersucht.

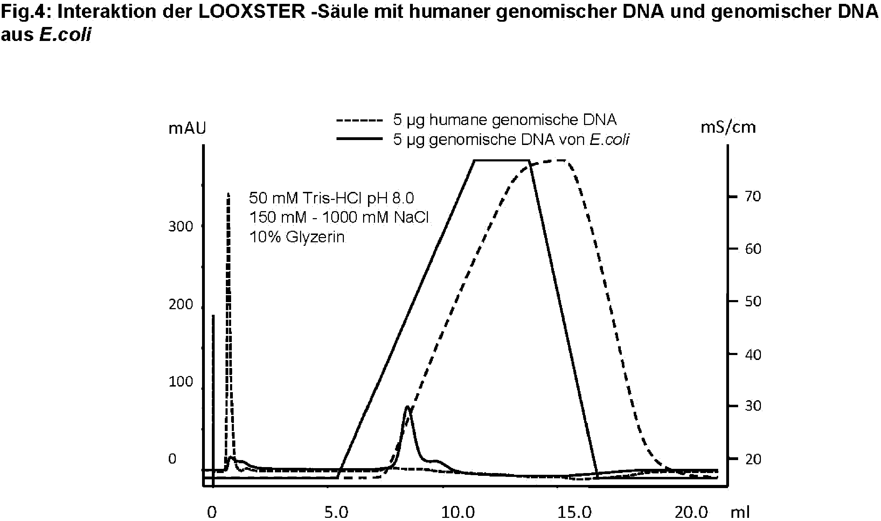

Das Experiment zeigt, dass unter den angegebenen experimentellen Bedingungen die LOOXSTER-Proteine fähig sind, wirksam an bakterielle ausschließlich nichtmethylierte CpG-Dinukleotide enthaltende DNA zu binden, jedoch nicht signifikant mit methylierte CpG-Dinukleotide enthaltender humaner genomischer DNA interagieren können (vgl. Fig. 4).

Insbesondere simuliert Fig. 4 die Bindungsverhältnisse für eine Mischung aus humaner und bakterieller DNA. Die chromatographischen Bindungsstudien gemäß Fig. 4 wurden jeweils getrennt mit humaner und E.coli genomischer DNA (jeweils 5 µg) durchgeführt und die Daten wurden zusammen in Fig. 4 gegen das Elutionsvolumen aufgetragen. Unter den vorliegenden experimentellen Bedingungen (50 mM Tris--HCl, pH 8.0, 150-1000 mM NaCl) bindet das LOOXSTER-Protein und insbesondere das CXXC1-p181 nichtmethylierte genomische DNA aus E.coli nahezu quantitativ, während keine signifikanten Wechselwirkungen mit humaner genomischer DNA auftreten. Dies ist daran zu erkennen, dass die größtenteils methylierte CpG-Dinukleotide enthaltende humane DNA als scharfer Peak im Säulendurchlauf erscheint, während die ausschließlich nichtmethylierte CpG-Dinukleotide enthaltende bakterielle DNA aus E. coli erst nach Anlegung einer bestimmten Salzkonzentration von der Säule eluiert wird.

Es ist dem Fachmann wohlbekannt, dass zur Elution einer nichtmethylierte CpG-Dinukleotide enthaltenden DNA - unabhängig von ihrer Herkunft - von einem LOOXSTER-Protein, z.B. CXXC1-p181, auch andere Elutionsmittel verwendet werden können. So können auch beispielsweise weitere gut wasserlösliche und nicht radioaktive Alkali- und Erdalkalimetallsalze als Elutionsmittel in Betracht kommen.

### Methylierungsabhängigkeit der LOOXSTER-Protein/DNA-Wechselwirkung

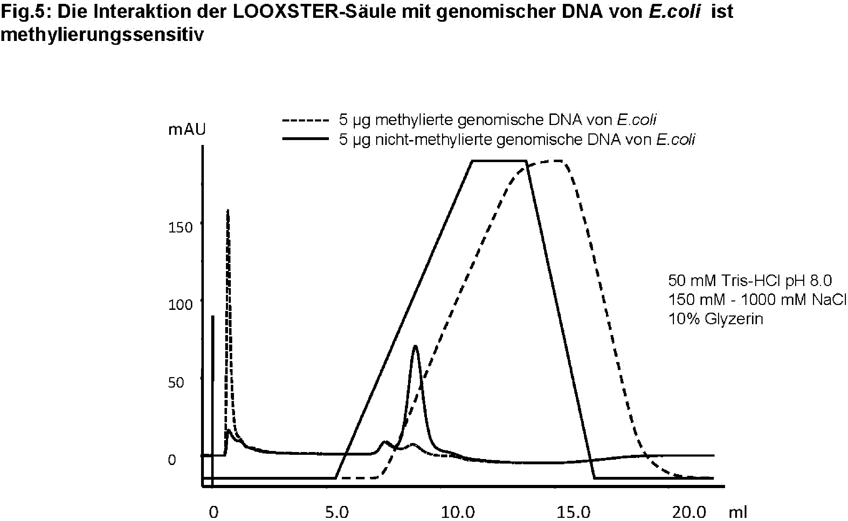

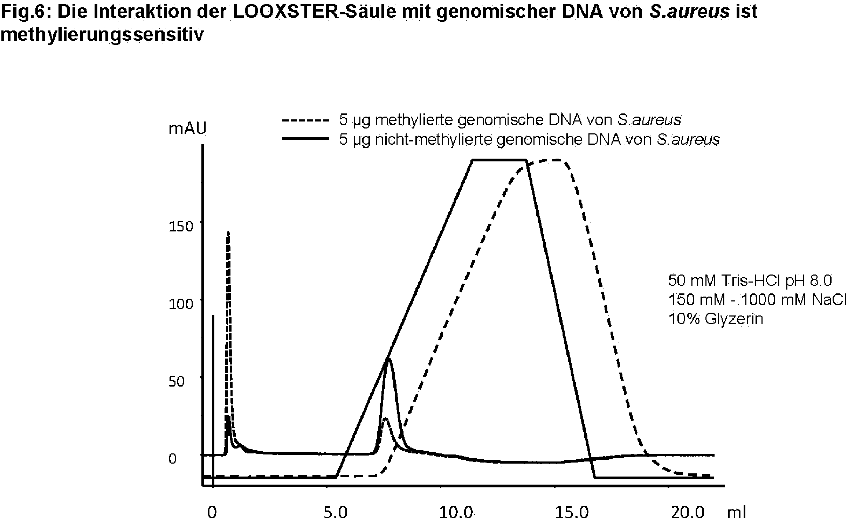

CXXC-haltige Proteine im Allgemeinen und insbesondere die erfindungsgemäß verwendeten LOOXSTER-Proteine sind - wie schon mehrfach erwähnt - Proteine, die spezifisch an nichtmethylierte CpG-Dinukleotide innerhalb einer DNA binden können. Um die Abhängigkeit der Wechselwirkung zwischen den erfindungsgemäßen LOOXSTER-Proteinen und DNA von deren Methylierungsgrad zu zeigen, wurden im Rahmen der vorliegenden Anmeldung folgende Bindungsstudien durchgeführt: Auf einer Sepharose-Säule mit daran immobilisiertem LOOXSTER-Protein wurde eine Affinitätschromatographie wie z.B. in Fig. 4 durchgeführt. Hierfür wurde DNA aus zwei unterschiedlichen Bakterienspezies, nämlich E. coli einerseits und S. aureus andererseits isoliert. Die speziesspezifische DNA wurde dann *in vitro* künstlich mittels Sss I Methyltransferase methyliert und die jeweilige speziesspezifische methylierte DNA und die entsprechende unveränderte native DNA getrennt auf eine LOOXSTER-Affinitätssäule aufgetragen. Die Chromatogramme für die native und die *in vitro* methylierte bakterielle DNA sind für E. coli in Fig. 5 gezeigt. Für S. aureus sind die Ergebnisse in Fig. 6 gezeigt. Für beide Bakterienspezies gilt, dass unter den gegebenen experimentellen Bedingungen die jeweilige native nichtmethylierte DNA von der LOOXSTER-Säule gebunden wird, während die künstlich *in vitro* methylierten E. coli und S. aureus DNA-Präparationen nahezu vollständig im Säulendurchlauf erscheinen.

### Abhängigkeit der LOOXSTER-Protein/DNA-Wechselwirkung von der Häufigkeit des CpG-Motivs innerhalb der DNA

Eine LOOXSTER-Säule, die mit 1 ml Ni Sepharose, an die 1 mg nichtmethylierte CpG-Motive bindendes Protein immobilisiert wurde, gefüllt ist, enthält ca. 2,5x10¹⁶ Proteinmoleküle. In einem LOOXSTER-Chromatographieassay, in dem 5 µg E.coli DNA mit 9,84x10⁸ Genomkopien eingesetzt werden, ist das stöchiometrische Verhältnis zwischen Protein- und DNA-Molekülen ca. 2,6x10⁷:1. Die Bindungsstöchiometrie und die Tatsache, dass ein einzelnes DNA-Molekül typischerweise zahlreiche CpG-Bindungsstellen aufweist, bestätigt die in der vorliegenden Anmeldung vertretene These, dass ein einziges DNA-Molekül mehrere Wechselwirkungen mit an nichtmethylierte CpG-Motive bindenden Proteinen eingehen kann.

CpG-Dinukleotide sind nicht gleichförmig innerhalb der genomischen DNA verteilt. Diese Tatsache macht sich die vorliegende Erfindung zu Nutze. Es ist somit möglich, dass sich DNA, welche reich an nichtmethylierten CpG-Motiven ist, in stärkerem Maße an einen Träger mit entsprechenden Affinitätsliganden für nichtmethylierte CpG-Motive bindet, als DNA, die arm an nichtmethylierten CpG-Motiven ist.

Allerdings ist es aus dem Stand der Technik bekannt, dass unterschiedliche CpG-Dinukleotide innerhalb einer einzigen DNA-Sequenz unter Umständen nicht mit gleicher Affinität an CXXC-Domänen binden (vgl. Lee JH, Voo KS, Skalnik DG (2001): Identification and characterization of the DNA binding domain of CpG-binding protein. J Biol Chem. Nov 30;276(48):44669-76. Epub 2001 Sep 25).

Insbesondere fanden Lee et al. (2001) heraus, dass beim hCGP Protein die flankierenden Nukleotide der CpG-Dinukleotide eine modulierende Rolle für die Bindungsaffinität spielten. Insbesondere wurde von diesen Autoren festgestellt, dass solche CpG-Motive, die von Adenin und Thymin flankiert werden, die höchste Bindungsaffinität zeigten. Das sich ergebende Motiv ist das sogenannte CpG-Consensus- Motiv mit der Sequenz: A/C CG A/C).

Sowohl der genomische GC-Gehalt wie auch die Häufigkeit der CpG- Motive schwanken stark zwischen den einzelnen Mikroorganismenspezies.

Im Rahmen der vorliegenden Erfindung wurde deshalb die Bindungsaffinität des LOOXSTER-Proteins gegenüber unterschiedlichen Bakteriengenomen mit unterschiedlichen GC-Gehalten und CpG- Motivhäufigkeiten untersucht.

Hierbei hat sich herausgestellt, dass es, wenigstens für die in Tabelle 2 wiedergegebenen Bakterienspezies eineindeutig möglich ist, die elektrische Leitfähigkeit von Fraktionen, die von einer Säule aus an einen festen Träger immobilisierten an nichtmethylierte CpG-Motive bindenden Proteinen mit einer bestimmten Salzkonzentration eluiert wurden ("Elutionsleitfähigkeit"), mit der Häufigkeit des CpG-Vorkommens in einer DNA zu korrelieren. Damit ist mittels einer geeigneten Kalibration eine Zuordnung eines bestimmten Leitfähigkeitswertes über die CpG-Häufigkeit und/oder des GC-Gehaltes der zu untersuchenden DNA-Moleküle und die Bestimmung der Bakterienspezies möglich. Sofern eine bekannte Bakterienspezies der Zielorganismus ist, kann zur Kalibrierung ein Datenbankeintrag oder eine Literaturangabe zum GC-Gehalt und/oder der CpG-Häufigkeit verwendet werden. Für den Fall, dass es sich um einen Mikroorganismus handelt, der noch nicht in den Datenbanken bezüglich GC-Gehalt und/oder CpG-Häufigkeit charakterisiert ist, können diese Daten einerseits selbst anhand dem Fachmann wohl bekannter Sequenzierverfahren, Bestimmung der Schmelztemperatur oder mit Hilfe der Gaschromatographie oder Gleichgewichtszentrifugation erfaßt werden oder es kann eine Kalibration mit anderen Bakterien-DNAs erfolgen, die eine eineindeutige Zuordnung des Zielorganismus zur Leitfähigkeit als Maß für die Konzentration des Elutionsmittels, insbesondere eines Salzgradienten, erlauben.

**Tabelle 2**

| CpG-Motivhäufigkeiten und Korrelation mit der Elutionsleitfähigkeit als Maß für die Konzentration eines Elutionsmittels für einige beispielhafte Bakterienspezies | | | | |
|---|---|---|---|---|
| Spezies | 1/CpG-Motiv-Häufigkeit [nt] | GC-Gehalt [%] | Genomgröße [Mbp] | Elutionsleitfähigkeit [mS/cm] |
| Pseudomonas aeroginosa | 36.2 | 66.3-66.6 | 6.59 | 31.733 |
| Stenotrophomonas maltophilia | 36.6 | 66.3 | 4.85 | 31.463 |
| Neisseria meningitidis | 42.8 | 51.5-51.9 | 2.15 | 29.888 |
| Escherichia coli | 55.5 | 50.4-50.9 | 4.75 | 26.742 |
| Proteus mirabilis | 112.1 | 38,9 | 4,06 | 20,63 |
| Acinetobacter baumannii | 121,4 | 38.9-39.4 | 3,98 | 21.272 |
| Streptococcus pneumoniae | 145.0 | 39.5-39.8 | 2.21 | 17.729 |
| Staphylococcus aureus | 159.7 | 32.8-33.0 | 2.81 | 18.047 |
| Clostridium perfringens | 803.7 | 28.2-28.6 | 3.26 | 18.056 |

In Tabelle 2 sind die reziproken CpG-Consensus Motivhäufigkeiten, GC-Gehalte, Genomgrößen sowie die gemessene Elutionsleitfähigkeit für eine Reihe von beispielhaften Mikroorgansimen gezeigt.

Hierbei bedeuten die Zahlen der reziproken CpG-Motivhäufigkeiten in Tabelle 2, dass jeweils ein CpG-Motiv für die in Spalte 2 angegebene Nukleotidzahl [nt] auftritt.

Für Pseudomonas aeruginosa bedeutet dies, dass 1 CpG-Motiv auf 36,2 Nukleotide seines Genoms entfällt. Für Clostridium perfringens beträgt dieser Wert lediglich 1 CpG-Motiv auf 803,7 Nukleotide des C. perfringens-Genoms.

Die im Rahmen der vorliegenden Erfindung erhaltenen Ergebnisse belegen eindeutig, dass die Affinität der LOOXSTER-Proteine zu Ziel-DNAs aus Mikroorganismen - in Form der dargelegten Affinitätschromatographie mit Salzgradientenelution - bestimmt wird durch den Gehalt an CpG-Motiven, insbesondere CpG-Consensus Motiven, die in der DNA des jeweiligen Mikroorganismus enthalten sind.

Damit kann zusammenfassend festgestellt werden, dass die CpG-Häufigkeit mit der Bindungsaffinität von immobilisiertem an nichtmethylierte CpG-Dinukleotide bindendem Protein (immobilisiertem LOOXSTER-Protein) korreliert werden kann und die Zuordnung der eluierten Fraktionen nach Ionenstärke, bzw. Konzentration des Elutionsmittels erfolgen kann.

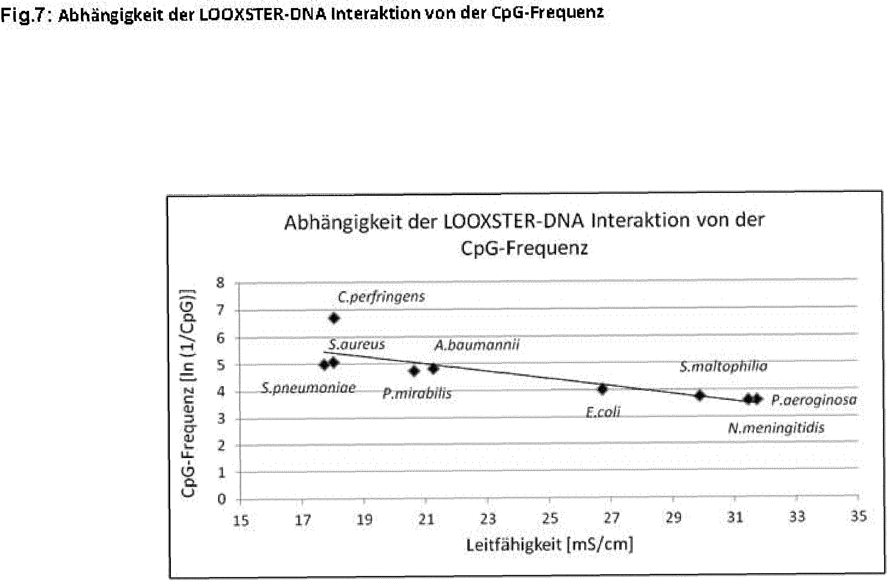

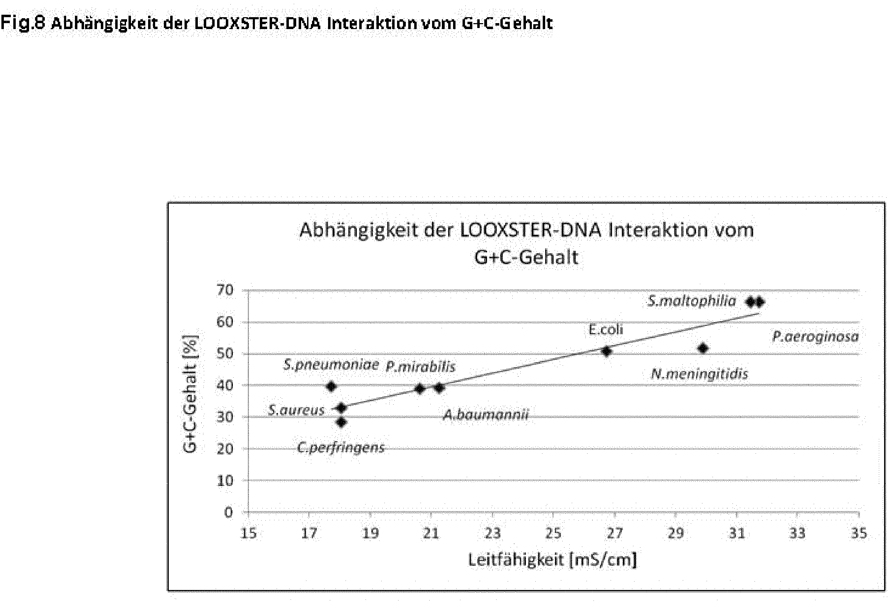

Ein weiterer Beweis hierfür ist in Fig. 7 und Fig. 8 gegeben. In Fig. 7 ist der natürliche Logarithmus (Ln) der reziproken CpG-Häufigkeit gemäß Tabelle 2 gegen die Leitfähigkeit als Maß für die Ionenstärke bzw. die Konzentration der jeweils eluierten Fraktionen sowie die dazugehörigen Spezies aufgetragen, wobei ein Unterschied von 10 mM NaCl etwa einer Leitfähigkeitsdifferenz von ca. 1,11 mS/cm entspricht. Mit anderen Worten gibt die Abszisse in Fig. 7 an, welche Salzkonzentration (ausgedrückt als Leitfähigkeit) erforderlich ist, um eine an eine Matrix mit immobilisiertem an nichtmethylierte CpG-Motive bindendem Protein (immobilisiertem LOOXSTER-Protein) gebundene DNA zu eluieren.

In Fig. 8 ist der GC-Gehalt gemäß Tabelle 2 gegen die Leitfähigkeit als Maß für die Ionenstärke bzw. die Konzentration der jeweils eluierten Fraktionen sowie die dazugehörigen Spezies aufgetragen, wobei ein Unterschied von 10 mM NaCl etwa einer Leitfähigkeitsdifferenz von ca. 1,11 mS/cm entspricht. Mit anderen Worten gibt die Abszisse in Fig. 8 an, welche Salzkonzentration (ausgedrückt als Leitfähigkeit) erforderlich ist, um eine an eine Matrix mit immobilisiertem an nichtmethylierte CpG-Motive bindendendem Protein (immobilisiertem LOOXSTER-Protein) gebundene DNA zu eluieren.

Unter den gegebenen experimentellen Bedingungen (Leitfähigkeit des Bindungspuffers: 16,325 mS/cm) zeigt im gegebenen Beispielsfall des LOOXSTER-Proteins humane DNA keine Affinität zu der LOOXSTER-Säulenmatrix. Ebenso konnte ein Einfluß der unterschiedlichen Genomgrößen der Bakterienspezies auf die LOOXSTER-DNA-Interaktion experimentell weitgehend ausgeschlossen werden.

Demzufolge ist das erfindungsgemäße Verfahren geeignet, unterschiedliche DNA-Spezies, die gleichzeitig in einer Probe vorliegen, aufgrund ihres unterschiedlichen Gehaltes an nichtmethylierten CpG-Dinukleotiden und ihres unterschiedlichen Methylierungsgrades zu trennen, zu erfassen oder anzureichern.

Das erfindungsgemäße Verfahren kann beispielsweise zur Erregeridentifizierung bei Patienten mit Infektionen verwendet werden. Eine besondere Rolle kommt dem Verfahren bei der Erregerbestimmung bei Sepsispatienten zu.

## Patentansprüche

1. In vitro Verfahren zum Trennen, Erfassen oder Anreichern von unterschiedlichen DNA-Spezies, die gleichzeitig in einer Probe vorliegen, aufgrund von mit unterschiedlicher Häufigkeit auftretenden nichtmethylierten CpG-Dinukleotiden, innerhalb jeder vorliegenden DNA-Spezies, wobei nichtmethylierte CpG-Dinukleotide solche sind, welche keine Methylgruppe in Position 5 des Cytosins aufweisen, wobei
die DNA, welche mehrere unterschiedliche DNA-Spezies enthält, aus der Probe isoliert wird und eine wässrige Lösung der isolierten DNA hergestellt wird;
die DNA-Lösung mit wenigstens einem an einem Trägermaterial immobilisierten an nichtmethylierte CpG-Dinukleotide bindenden Protein in Kontakt gebracht wird;
unspezifisch gebundene DNA und methylierte DNA ausgewaschen wird; und
das Trägermaterial mit wenigstens einem Elutionsmittel, welches unterschiedliche Konzentrationen aufweist, eluiert wird, wobei bestimmte Konzentrationsbereiche des eingesetzten Elutionsmittels mit bestimmten CpG-Dinukleotid-Häufigkeiten in den vorliegenden DNA-Spezies korrelieren, so dass während der Elution unterschiedliche Fraktionen von DNA-Spezies mit unterschiedlichen Häufigkeiten an CpG-Dinukleotiden erhalten werden, wobei die unterschiedlichen DNA-Spezies aus unterschiedlichen Bakterien und/oder Pilzen stammen.

2. Verfahren nach Anspruch 1, **dadurch gekennzeichnet, dass** die Bakterien und/oder Pilze insbesondere ausgewählt sind aus der Gruppe bestehend aus:
den Bakteriengattungen:
Pseudomonas; Stenotrophomonas; Neisseriaceae; Echerichia; Proteus; Acinetobacter; Streptococcus; Staphylococcus; Clostridium;
den Bakterienspezies:
Pseudomonas aeroginosa, Stenotrophomonas maltophila, Neisseria meningitidis, Echerichia coli, Proteus mirabilis, Acinetobacter baumannii,
Streptococcus pneumoniae, Staphylococcus aureus, Clostridium perfringens;
den Pilzgattungen Aspergillus und Saccharomyces; sowie
den Pilzspezies Aspergillus niger und Saccharomyces cerevisiae.

3. Verfahren nach Anspruch 1 oder 2, **dadurch gekennzeichnet, dass** das Erfassen von unterschiedlichen DNA-Spezies zur Identifikation von Mikroorganismen, insbesondere Bakteriengattungen und/oder Pilzgattungen und/oder Cyanobakteriengattungen und/oder Algengattungen und/oder und/oder Protozoagattungen und/oder Bakterienspezies und/oder Cyanobakterienspezies und/oder Algenspezies und/oder Pilzspezies und/oder Protozoaspezies verwendet wird.

4. Verfahren nach Anspruch 3, **dadurch gekennzeichnet, dass** die Identifikation von Mikroorganismen anhand von erfassten DNA-Spezies in der Probe über folgende Zuordnung von Häufigkeiten des CpG-Dinukleotids innerhalb der erfassten DNA-Spezies erfolgt, ausgedrückt als Kehrwert der Frequenz (v) der CpG-Dinukleotide, wobei die Frequenz die Anzahl der Nukleotide [nt] in einer DNA Spezies bedeutet, auf die statistisch ein CpG-Dinukleotid entfällt:
| **Mikroorganismus-Spezies** | **1/ν CpG-Dinukleotide [nt]** |
|---|---|
| Pseudomonas aeruginosa | 36.0-36.4, bevorzugt 36.2 |
| Stenotrophomonas maltophilia | 36.5-36.8, bevorzugt 36.6 |
| Neisseria meningitidis | 42.4-43.2, bevorzugt 42.8 |
| Echerichia coli | 55.1-55.9, bevorzugt 55.5 |
| Proteus mirabilis | 111.5-112.6, bevorzugt 112.1 |
| Acinetobacter baumannii | 120.9-121.9, bevorzugt 121.4 |
| Streptococcus pneumoniae | 144.5-145.5, bevorzugt 145.0 |
| Staphylococcus aureus | 159.2-160.2, bevorzugt 159.7 |
| Clostridium perfringens | 801.7-805.7, bevorzugt 803.7 |

5. Verfahren nach einem der vorhergehenden Ansprüche, **dadurch gekennzeichnet, dass** als Maß für die Häufigkeit des CpG-Dinukleotids in einer DNA-Spezies der Gehalt an den Nukleobasen Guanin und Cytosin (GC-Gehalt) verwendet wird, ausgedrückt in % als $\left[\frac{mGuanin + mCytosin}{mAdenin + mThymin + mGuanin + mCytosin}\right] \times 100$
wobei m die Masse der jeweiligen Nukleobase bezeichnet.

6. Verfahren nach einem der vorhergehenden Ansprüche, **dadurch gekennzeichnet, dass** das Trägermaterial mit einer Salzlösung, insbesondere einem Salzgradienten, bevorzugt einem (NH₄)₂CO₃-Gradienten oder einem NaCl-Gradienten mit einer NaCl-Konzentration von 0,01 M bis 1,0 M, insbesondere 0,1 M bis 0,8 M, bevorzugt 0,2 M bis 0,7 M eluiert wird, wobei bestimmte Konzentrationsbereiche einer bestimmten CpG-Häufigkeit und/oder einem bestimmten GC-Gehalt entsprechen, so dass einzelne DNA-Fraktionen mit unterschiedlichen CpG-Häufigkeiten und/oder unterschiedlichen GC-Gehalten erhalten werden; und
Vergleichen der Salzkonzentrationen der einzelnen eluierten DNA-Fraktionen mit der Salzkonzentration von eluierter DNA mit bekannter CpG-Häufigkeit und/oder bekanntem GC-Gehalt aus bekannten Mikroorganismen, um den unbekannten Mikroorganismus anhand seiner CpG-Häufigkeit und/oder seines GC-Gehaltes in der jeweiligen DNA-Fraktion zu identifizieren.

7. Verfahren nach Anspruch 6, **dadurch gekennzeichnet, dass** die Salzkonzentration der einzelnen DNA-Fraktionen über eine Messung der elektrischen Leitfähigkeit erfasst wird.

8. Verfahren nach einem der vorhergehenden Ansprüche, **dadurch gekennzeichnet, dass** das an nichtmethylierte CpG-Dinukleotide bindende Protein ausgewählt wird aus der Gruppe bestehend aus: CXXC-Domäne enthaltende Proteine; Proteinfragmente, welche wenigstens eine CXXC-Domäne aufweisen, insbesondere humanes nichtmethyl-CpG bindendes Protein CXXC1/CGBP sowie dessen Isoformen 1 und 2 und Fragmente davon, die die CXXC-Domäne enthalten; CXXC-Domäne enthaltende Fusionsproteine.

9. Verfahren nach Anspruch 8, **dadurch gekennzeichnet, dass** das an nichtmethylierte CpG-Dinukleotide bindende Protein an seinem N- und/oder C-terminalen Ende einen metallionenbindenden Linker, insbesondere ein Hisₙ-tag, aufweist, wobei n 3 bis 10, bevorzugt 6, ist.

10. Verfahren nach einem der vorhergehenden Ansprüche, **dadurch gekennzeichnet, dass** das Trägermaterial ein Metallchelat-bildendes Material ist, insbesondere eine Metallchelat-bildende Agarose, bevorzugt eine nickelionenbindende Agarose, vorzugsweise Sepharose, ist.

11. Verfahren nach Anspruch 10, **dadurch gekennzeichnet, dass** das an nichtmethylierte CpG-Dinukleotide bindende Protein an seinem N- und/oder C-terminalen Ende ein His₆- tag, aufweist, und über Ni²⁺-Kationen an Nickelchelat-bildende Sepharose immobilisiert ist.
